# FASCICULE DE BREVET EUROPEEN

(11) **EP 2 303 830 B1**
(45) Date de publication et mention de la délivrance du brevet: **19.02.2014**
(21) Numéro de dépôt: 09772647.5
(22) Date de dépôt: 05.06.2009
(51) Int. Cl.: C07C 231/02, C07C 233/51, C07C 235/34, C12P 13/00, A61K 31/165, A61K 8/44, A61P 39/06, A61Q 90/00

(54) **PROCEDE MICROBIOLOGIQUE DE SYNTHESE DE DERIVES CINNAMOYL AMIDES D'ACIDES AMINES**
MIKROBIOLOGISCHES VERFAHREN ZUR SYNTHESE VON CINNAMOYLAMIDDERIVATEN VON AMINOSÄUREN
MICROBIOLOGICAL METHOD FOR SYNTHESIZING CINNAMOYL AMIDE DERIVATIVES OF AMINO ACIDS

(30) Priorité: 09.06.2008 FR 0803188
(43) Date de publication de la demande: 06.04.2011
(73) Titulaire: Institut de Recherche pour le Développement, 13572 Marseille Cedex 02 (FR)
(72) Inventeur: LORQUIN, Jean, F-83330 Le Beausset (FR)
(74) Mandataire: Mena, Sandra
(86) Numéro de dépôt international: PCT/FR2009/000662
(87) Numéro de publication internationale: WO 2010/000964

(56) Documents cités:
- US-A- 5 212 158
- SONG KYUNG-SIK: "Exogenous D-Ala enhances the accumulation of p-coumaroylamino acids in Ephedra distachya cultures" ARCHIVES OF PHARMACAL RESEARCH, NATL. FISHERIES UNIVERSITY, PUSAN, KR, vol. 18, no. 5, 1 janvier 1995 (1995-01-01), pages 336-339, XP008099922 ISSN: 0253-6269
- TU G ET AL: "Novel aminopeptidase N inhibitors derived from 1,3,4-thiadiazole scaffold" BIOORGANIC & MEDICINAL CHEMISTRY, ELSEVIER SCIENCE LTD, GB, vol. 16, no. 14, 5 juin 2008 (2008-06-05), pages 6663-6668, XP022941531 ISSN: 0968-0896 [extrait le 2008-06-05]
- SONG KYUNG SIK ET AL: "N-Acylamino acids from Ephedra distachya cultures" PHYTOCHEMISTRY, PERGAMON PRESS, GB, vol. 31, no. 3, 1 janvier 1992 (1992-01-01), pages 823-826, XP002508849 ISSN: 0031-9422
- LEE ET AL: "Caffeoylglycolic and caffeoylamino acid derivatives, halfmers of l-chicoric acid, as new HIV-1 integrase inhibitors" EUROPEAN JOURNAL OF MEDICINAL CHEMISTRY, EDITIONS SCIENTIFIQUE ELSEVIER, PARIS, FR, vol. 42, no. 10, 13 octobre 2007 (2007-10-13), pages 1309-1315, XP022297754 ISSN: 0223-5234
- MIMOTO T ET AL: "RATIONAL DESIGN AND SYNTHESIS OF A NOVEL CLASS OF ACTIVE SITE-TARGETED HIV PROTEASE INHIBITORS CONTAINING A HYDROXYMETHYLCARBONYL ISOSTERE. USE OF PHENYLNORSTATINE OR ALLOPHENYLNORSTATINE AS A TRANSITION-STATE MIMIC" CHEMICAL AND PHARMACEUTICAL BULLETIN, PHARMACEUTICAL SOCIETY OF JAPAN, TOKYO, JP, vol. 39, no. 9, 1 janvier 1991 (1991-01-01), pages 2465-2467, XP000653624 ISSN: 0009-2363
- KNOWLES H S ET AL: "Desulfonylation of Amides using Tributyltin Hydride, Samarium Diiodide or Zinc/Titanium Tetrachloride. A Comparison of Methods" TETRAHEDRON, ELSEVIER SCIENCE PUBLISHERS, AMSTERDAM, NL, vol. 56, no. 7, 1 novembre 2000 (2000-11-01), pages 979-988, XP004187697 ISSN: 0040-4020
- SAKAKURA, AKIRA ET AL: "Molybdenum Oxides as Highly Effective Dehydrative Cyclization Catalysts for the Synthesis of Oxazolines and Thiazolines" ORGANIC LETTERS, 7(10), 1971-1974 CODEN: ORLEF7; ISSN: 1523-7060, 2005, XP002551211
- DATABASE CA [Online] CHEMICAL ABSTRACTS SERVICE, COLUMBUS, OHIO, US; NAKANISHI, KAZUHIRO ET AL: "A novel aminoacylase from Streptomyces mobaraensis that efficiently catalyzes hydrolysis/synthesis of N-acyl-L-amino acids and N-acyl-peptides" XP002508850 extrait de STN Database accession no. 2006:236619 -& JP 2006 067870 A (TSUNO FOOD INDUSTRIAL CO., LTD., JAPAN; OKAYAMA UNIVERSITY) 16 mars 2006 (2006-03-16)
- LEE, SANGKU ET AL: "Hydroxylated hydrocinnamides as hypocholesterolemic agents" BULLETIN OF THE KOREAN CHEMICAL SOCIETY , 28(10), 1787-1791 CODEN: BKCSDE; ISSN: 0253-2964, 2007, XP002508847
- KIM, SOON-JA ET AL: "Anticholesterolemic effect of 3,4-di(OH)-phenylpropionic amides in high- cholesterol fed rats" TOXICOLOGY AND APPLIED PHARMACOLOGY , 208(1), 29-36 CODEN: TXAPA9; ISSN: 0041-008X, 2005, XP002508848
- DATABASE CA [Online] CHEMICAL ABSTRACTS SERVICE, COLUMBUS, OHIO, US; KATO, HISATOYO ET AL: "Preparation of N-(carboxyalkyl)dihydoferulamides and their uses as antioxidants and tyrosinase inhibitors" XP002508851 extrait de STN Database accession no. 2006:169901 -& JP 2006 052150 A (TOA GOSEI CHEMICAL INDUSTRY CO., LTD., JAPAN) 23 février 2006 (2006-02-23)
- DATABASE CA [Online] CHEMICAL ABSTRACTS SERVICE, COLUMBUS, OHIO, US; KATO, HISATOYO ET AL: "Preparation of amides of dihydroferulic acid with L-.alpha.-amino acids and their uses as antioxidants and tyrosinase inhibitors" XP002508852 extrait de STN Database accession no. 2006:166841 -& JP 2006 052152 A (TOA GOSEI CHEMICAL INDUSTRY CO., LTD., JAPAN) 23 février 2006 (2006-02-23)
- DATABASE CA [Online] CHEMICAL ABSTRACTS SERVICE, COLUMBUS, OHIO, US; ANAI, MAKIKO ET AL: "Cinnamic acid derivatives as tyrosinase inhibitors and antioxidants , and their uses" XP002508853 extrait de STN Database accession no. 2004:507814 -& JP 2004 175778 A (SOGO PHARMACEUTICAL CO., LTD., JAPAN) 24 juin 2004 (2004-06-24)
- SONG KYUNG-SIK ET AL: "p-Coumaroylamino acids from yeast -elicited Ephedra distachya cultures" ARCHIVES OF PHARMACAL RESEARCH, NATL. FISHERIES UNIVERSITY, PUSAN, KR, vol. 17, no. 1, 1 janvier 1994 (1994-01-01), pages 48-50, XP008099923 ISSN: 0253-6269
- DATABASE CA [Online] CHEMICAL ABSTRACTS SERVICE, COLUMBUS, OHIO, US; TADA, TAKAHIRO ET AL: "Compositions containing caffeic acid amides for skin-lightening cosmetics and topical preparations" XP002508854 extrait de STN Database accession no. 2003:525333 -& JP 2003 192522 A (MIKIMOTO PHARMACEUTICAL CO., LTD., JAPAN) 9 juillet 2003 (2003-07-09)

## Description

L'invention a pour objet un procédé de production microbiologique de dérivés de cinnamoyl amides d'acides aminés, certains produits en résultant et leurs utilisations, notamment comme antioxydants.

Des molécules de cette famille ont été essentiellement isolées du règne végétal, principalement dans l'organe reproducteur de plantes, alors qu'elles semblent être absentes des parties vertes de celles-ci (Martin-Tanguy et al., Phytochemistry, 17: 1927-1928, 1978 ; FEBS Lett., 108: 176-178, 1979). En 2003, Alemano et al. (Annals of Botany, 92: 613-623) ont identifié la N-caffeoyl-tyrosine et la N-cafféoyl-DOPA dans des cellules d'embryogénèse somatique de *Theobroma cacao.* Plus tard, dans les grains de café vert (robusta), la *p*-coumaroyl-N-tyrosine, la féruloyl-N-tyrosine, la féruloyl-N-tryptophane et la cafféoyl-N-phénylalanine ont été mises en évidence (Clifford and Knight, Food chemistry, 87: 457-453, 2003), alors que leur pourvoir antioxydant avait déjà été constaté auparavant (Sanbongi et al., J. Agric. Food Chem. 46: 454-457, 1998). Puis, Stark et Hoffman (J. Agric. Food Chem., 53: 5419-5428, 2005) puis Stark et al. (J. Agric. Food Chem., 54: 2859-2867, 2006) ont ensuite identifié et complété la liste par d'autres analogues présents dans les mêmes sources végétales.

Les amides d'acides cinnamique et hydroxycinnamique ont été surtout répertoriés et évalués pour leurs activités antioxydantes importantes (Spasova et al., J. Peptide Sci., 12: 369-375, 2006), propriété qui semble inhérente à toute structure de ce type.

D'autres molécules comme le 4-hydroxycinnamoyl-(L-phénylalanine méthyl ester) amide ou encore le 3,4-dihydroxycinnamoyl-(L-aspartic acid dibenzyl ester) amide ont été cités pour leur activité antioxydante contre l'oxydation des LDL (low-density lipoproteins) par le cuivre, et ces molécules inhibent en même temps l'activité de l'acyl-CoA:cholesterol acyltransférase humaine (Lee et al., Biorg. Med. Chem. Letters, 14: 4677-4681, 2004); elles sont donc impliquées dans l'hypercholestérolémie. Les N-cinnamoyl amides ont également été décrits comme étant des agents anti-agrégants plaquettaires (Hung et al., Bioorganic and Medicinal Chemistry, 13: 1791-1797, 2005). Les clovamides (N-cinnamoyl tyrosine) qui sont des phytoalexines s'accumulant dans les plantes suite à l'attaque de pathogènes (Yamamoto et al., Pharmacol Biochem Behav, 40: 465-469 , 1991) en font également partie, et sont à ce titre connus pour être des antimicrobiens puissants.

Enfin des activités vaso-relaxantes de dérivés N-cafféoylamino R acides (R = GABA ou acide hexanoique) ont aussi été décrits récemment (Iizuka et al., Yakugaku Zasshi, 123: 963-971, 2003).

Les dérivés cinnamoyl amide d'acides aminés constituent donc une famille de molécules au potentiel important aussi bien dans le domaine thérapeutique qu'en cosmétique, ou encore comme agent conservateur. Toutefois, leur isolement à partir de plantes est difficile, les rendements et les quantités sont faibles, et les structures peu diversifiées.

Des méthodes pour la synthèse de certaines de ces molécules par voie chimique ont été décrites ; mais elles requièrent plusieurs étapes, utilisent des composés toxiques et sont par conséquent difficiles à industrialiser :
Dans la synthèse décrite par Iizuka et al. (Yakugaku Zasshi, 123: 963-971, 2003), quatre étapes sont nécessaires : **(1)** protection des fonctions hydroxyles du cycle par le chloro-acétate d'éthyle en milieu basique, **(2)** puis couplage par le dicyclohexylcarbodiimide (DCCDI); **(3)** l'acide aminé est ensuite couplé en présence de triéthylamine, et enfin **(4)** une hydrolyse libère les fonctions hydroxyle du noyau aromatique (dé-protection). Par cette méthode ont été synthétisées la N-cafféoylglycine, la N-cinnamoylglycine, la N-cafféoyl-β-alanine, la N-cinnamoyl-β-alanine.

Une autre synthèse plus rapide basée sur la fixation de l'acide aminé (acide L-aspartique ou L-tryptophane dans ce cas) sur le chlorure d'acyle correspondant après protection préalable des groupes hydroxyles du cycle aromatique par la diméthylaminopyridine (DMAP). La réaction d'amidation s'effectue par reflux en présence de tétrahydrofurane (THF), suivi enfin d'une dé-protection pour libérer les hydroxyles (Stark et Hofmann, J. Agric. Food Chem., 53: 5419-5428, 2005; Stark et al, J. Agric. Food Chem., 54: 2859-2867, 2006).

Citons également les travaux de Tada et al. (J. Oleo Sci., 51: 19-27, 2002) qui décrivent la synthèse de différents dérivés dont l'ester méthylique de la N-cafféoyl-O-acétylsérine qui a une forte activité antioxydante et inhibitrice de la tyrosinase. Par ailleurs, Lee et al. (Bioorg. Med. Chem. Lett., 14: 4677-4681, 2004) ont effectué la synthèse d'esters de *p*-coumaroyl amide de l'acide aspartique (ester méthylique) et de la phénylalanine (ester dibenzylique) respectivement, par une méthode équivalente à Iizuka et al. (Yakugaku Zasshi, 123: 963-971, 2003) (voir plus haut).

Une synthèse utilisant une arylase de *Streptomyces mobaraensis* a également été décrite dans la Demande JP 2006-067870 A.

Il subsistait donc le besoin d'une méthode simple, facile à mettre en oeuvre, industrialisable, et permettant la préparation de dérivés cinnamoyl amide d'acides aminés. L'invention a pour premier objet un procédé de préparation de molécules répondant à la formule (I) suivante : 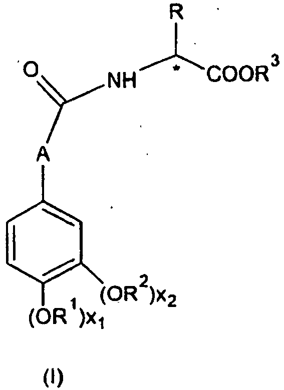 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre, représentent un groupement choisi parmi : H, les alkyles en C₁-C₆, le phényle, le benzyle, les groupements -CO-R⁴, R⁴ représentant un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L ou D de l'acide aminé -NH-CHR-COO-, * peut représenter l'une ou l'autre des configurations suivant le choix de R,
R représente une chaîne latérale d'acide aminé choisie parmi : -H (glycine), -CH₃ (L-alanine, D-alanine), -CH₂OH (L-sérine), -CHOH-CH₃ (L-thréonine), -CH₂OR⁵ (L-sérine protégée sur sa fonction hydroxyle), -CHOR⁵-CH₃ (L-thréonine protégée sur sa fonction hydroxyle), R⁵ représentant un groupement choisi parmi : H, les alkyles en C₁-C₆, le phényle, le benzyle, les groupements -CO-R⁶, R⁶ représentant un groupement choisi parmi : les alkyles en C₁-C₆, le phényle, le benzyle.

Par alkyle en C₁-C₆ on entend un groupement alkyle, linéaire ramifié ou cyclique, comprenant de 1 à 6 atomes de carbone, comme par exemple un groupement méthyle, éthyle, n-propyle, isopropyle, n-butyle, t-butyle, isobutyle, n-pentyle, n-hexyle.

Selon l'invention, les molécules de formule (I) sont préparées par un procédé qui comporte au moins une étape de culture d'au moins une bactérie choisie parmi les Bacillaceae, en présence d'au moins deux substrats sélectionnés parmi ceux de formules (II) et (III) ci-dessous : 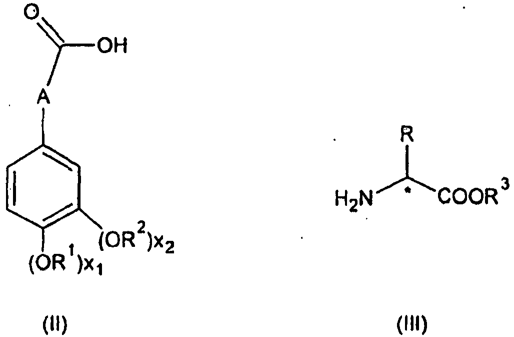
dans lesquelles A, x₁, x₂; R³,* et R ont la même définition que dans la formule (I),
R¹, R², indépendamment l'un de l'autre représentent un groupement choisi parmi : les alkyles en C₁-C₆, le phényle, le benzyle, les groupements -CO-R⁴, R⁴ représentant un groupement choisi parmi : les alkyles en C₁-C₆, le phényle, le benzyle.

Par alkyle en C₁-C₆ on entend un groupement alkyle, linéaire ramifié ou cyclique, comprenant de 1 à 6 atomes de carbone, comme par exemple un groupement méthyle, éthyle, n-propyle, isopropyle, n-butyle, t-butyle, isobutyle, n-pentyle, n-hexyle.

Avantageusement, le procédé de l'invention comporte une étape de culture d'au moins une bactérie choisie parmi celles appartenant au genre *Bacillus,* encore plus avantageusement une bactérie choisie parmi : *Bacillus subtilis, Bacillus licheniformis, Bacillus thermoamylovorans, Bacillus* (ou *Geobacillus*) *stearothermophilus, Bacillus caldotenax.*

Et préférentiellement, la bactérie est choisie parmi :
- *Bacillus subtilis* subsp. *Spizizenii* : DSM 15029 (température de croissance = 30°C)
- *Bacillus licheniformis* : DSM 13, ATCC 14580, NCIB - 9375 (température de croissance = 30°C)
- *Bacillus thermoamylovorans* (souche isolée par le laboratoire IRD-Marseille): DSM 13307 (température de croissance = 50°C)
- *Bacillus* (ou *Geobacillus*) *stearothermophilus* : DSM 22, ATCC 12980, NCIB 8923 (température de croissance = 55°C)
- *Bacillus caldotenax* : DSM 406 (température de croissance = 70°C)

De façon surprenante, l'inventeur a constaté que la culture d'une bactérie décrite ci-dessus en présence d'au moins un de chacun des substrats (II) et (III) donnait lieu à la formation d'une liaison amide entre la fonction acide du substrat (II) et la fonction amine du substrat (III) pour donner accès au produit de formule (I) correspondant. Le procédé de l'invention permet ainsi d'accéder à une famille de produits variés mais présentant néanmoins des caractéristiques structurales très spécifiques liées à la spécificité de la bactérie pour les substrats (II) et (III).

De façon préférentielle, dans la formule (I), l'une ou plusieurs des conditions ci-dessous sont satisfaites :
A représente un groupement -CH=CH-,
R¹, R², indépendamment l'un de l'autre représentent un groupement choisi parmi : H, -CH₃,
R³ représente H,
R représente une chaîne latérale d'acide aminé choisie parmi : -H (glycine), -CH₃ (L-alanine, D-alanine), -CH₂OH (L-sérine), -CHOH-CH₃ (L-thréonine),
En fonction de la façon dont on va utiliser le composé (I) ultérieurement, on choisit des précurseurs (II) et (III) appropriés :
Si le composé de formule (I) doit subir des étapes de synthèse ultérieures, il peut être souhaitable de le préparer sous une forme protégée, en choisissant les groupes protecteurs R¹, R², R³ et éventuellement R⁵ appropriés.

Notamment, lorsque x₁=1 ou x₂=1, alors, pour la mise en oeuvre de l'étape de culture, on choisit R¹≠H, respectivement R²≠H. En effet, la transformation en produit (I) est plus efficace lorsque le cycle aromatique du dérivé cinnamoyl (II) ne comporte pas de substituant ou que ceux-ci sont protégés. Ensuite, il est possible de libérer les fonctions hydroxyle présentes sur le cycle cinnamoyl par un traitement enzymatique avec une peroxydase (Paice et al., Applied Environmental Microbiology, 1993, 59:260-265).

Un composé de formule (I) comportant une chaîne peptidique R³ peut être obtenu directement par le choix d'un substrat correspondant (III) dans lequel la chaîne peptidique R³ est déjà présente, ou à partir d'un composé de formule (I) avec R³ = H par une étape ultérieure de greffage sur une chaîne peptidique ou de façon séquentielle par allongement de la chaîne d'acide aminé (Bodansky, Synthesis, 453-463, 1972).

Les composés de formule (I) avec A = -CH₂-CH₂- peuvent être obtenus directement à partir du précurseur (II) correspondant ou à partir d'un précurseur (II) dans lequel A représente -CH=CH- et en opérant, par des moyens bien connus de l'homme du métier, par une réduction chimique ou microbiologique de la double liaison (J March, Advanced Organic Chemistry, 1985, Wiley and sons, 691-700).

Lorsque l'on souhaite obtenir un seul composé de formule (I), on le prépare à partir des deux précurseurs correspondants (II) et (III). Il est possible de préparer un mélange de molécules de formule (I) par culture d'une bactérie décrite ci-dessus en présence d'un mélange de substrats (II) et/ou d'un mélange de substrats (III).

La culture de la bactérie est faite dans un milieu qui, outre les composés (II) et (III), comporte avantageusement des sels minéraux et un extrait de levure. De préférence, il comporte les sels minéraux suivants : KH₂PO₄, K₂HPO₄, NaCl, NH₄Cl. Avantageusement, le milieu de culture ne comporte pas d'autres acides aminés que ceux de formule (III), ce qui réduit les étapes de purification en fin de procédé.

Selon une autre variante préférée de l'invention, le milieu de culture comporte encore, outre le ou les acides aminés de formule (III), au moins un autre acide aminé choisi parmi : la lysine, la proline, la cystéine. Avantageusement, ces acides aminés sont sous forme (L). Ainsi que l'inventeur l'a constaté, ces acides aminés ne sont pas utilisés par la bactérie pour participer directement à la formation d'un produit de formule (I), mais ils contribuent à améliorer le rendement de synthèse des composés de formule (1) à partir d'acides aminés de formule (III) tels que définis ci-dessus présents dans le milieu. Avantageusement, la concentration d'un ou plusieurs de ces acides aminés dans le milieu de culture est comprise entre 1 et 4 mM.

Selon le procédé de l'invention, la concentration du composé de formule (II) dans le milieu de culture est préférentiellement comprise entre 0,5 et 10 mM, et avantageusement entre 1 et 5 mM.

Selon le procédé de l'invention, la concentration du composé de formule (III) dans le milieu de culture est préférentiellement comprise entre 0,5 et 20 mM, et avantageusement entre 2 et 8 mM.

Selon le procédé de l'invention, la proportion des composés de formule (II) et (III) en mole est : 0,30 < (II)/(III) < 0,9, de préférence 0,40 < (II)/(III) < 0,8, et avantageusement 0,50 < (II)/(III) < 0,65.

Selon une variante du procédé de l'invention, le précurseur (II) peut être introduit dans le milieu de culture sous forme d'un extrait végétal. En effet, l'acide cinnamique est un constituant de certains déchets agronomiques et de produits dérivés de l'industrie du bois. Il est possible dans le procédé de l'invention, lorsque (II) est l'acide cinnamique ou un dérivé de l'acide cinnamique, de l'introduire directement dans le milieu de culture sous forme d'une composition de déchets agronomiques ou d'effluents de l'industrie du bois. De tels produits doivent habituellement être retraités et, grâce au procédé de l'invention, sont ainsi valorisés.

Préférentiellement, le milieu de culture est un milieu pauvre en métaux. En particulier, le milieu de culture ne comporte pas de fer (notamment Fe²⁺ généralement fourni par FeSO₄ ou FeCl₂). En effet, il a été constaté que la présence de fer inhibait totalement la réaction de formation de (I).

A l'issue de la formation du composé de formule (I) dont la présence est contrôlée par HPLC et LC-MS, le produit est isolé du milieu de culture par un procédé qui comporte avantageusement les étapes suivantes :
- centrifugation,
- récupération du surnageant,
- purification du produit (I).

La purification peut être faite par tout moyen habituellement employé par l'homme du métier, tels que : extraction liquide-liquide au solvant, chromatographie, précipitation, cristallisation. Ces moyens sont illustrés de façon plus spécifique dans la partie expérimentale. Le procédé de l'invention a permis de synthétiser notamment les molécules suivantes :
- N-cinnamoyl-glycine,
- N-cinnamoyl-L-alanine,
- N-cinnamoyl-D-alanine,
- N-cinnamoyl-L-sérine,
- N-cinnamôyl-L-thréonine,
- N-(4-méthoxycinnamoyl)-glycine,
- N-(4-méthoxycinnamoyl)-L-alanine,
- N-(4-méthoxycinnamoyl)-D-alanine,
- N-(4-méthoxycinnamoyl)-L-sérine,
- N-(4-méthoxycinnamoyl)-L-thréonine
- N-(3,4-diméthoxycinnamoyl)-glycine,
- N-(3,4-diméthoxycinnamoyl)-L-alanine,
- N-(3,4-diméthoxycinnamoyl)-D-alanine,
- N-(3,4-diméthoxycinnamoyl)-L-sérine,
- N-(3,4-diméthoxycinnamoyl)-L-thréonine,
- N-coumaroyl-L-thréonine,
- N-cafféoyl-L-thréonine,
- N-coumaroyl-L-sérine
- N-cafféoyl-L-sérine
- N-coumaroyl-L-alanine,
- N-cafféoyl-L-alanine,
- N-coumaroyl-D-alanine,
- N-cafféoyl-D-alanine,
- N-coumaroyl-glycine,
- N-cafféoyl-glycine.

L'invention a encore pour objet un procédé de fabrication d'un médicament qui comporte au moins une étape de culture telle qu'elle a été décrite ci-dessus pour former un composé de formule (I).

L'invention a encore pour objet un procédé de préparation des composés répondant à la formule (Ia) ci-dessous : 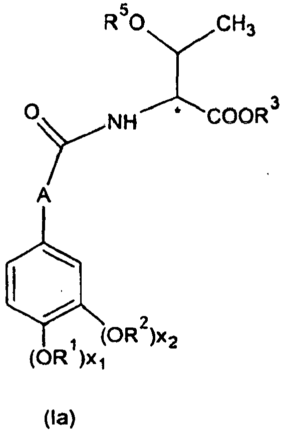 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L,
R⁵ représente un groupement choisi parmi : H, les alkyles en C₁-C₆, le phényle, le benzyle, les groupements -CO-R⁶ et R⁶ représente un groupement choisi parmi : les alkyles en C₁-C₆, le phényle, le benzyle.

De façon avantageuse, dans la formule (Ia), une ou plusieurs des conditions suivantes sont satisfaites :
A représente -CH=CH-,
x₁ est un entier choisi parmi 0 et 1,
x₂ est un entier choisi parmi 0 et 1,
R¹, R², indépendamment l'un de l'autre représentent un groupement choisi parmi : H, -CH₃,
R³ représente H,
* représente la configuration optique L,
R⁵ représente H.

De façon préférée, le composé de formule (Ia) est choisi parmi la liste suivante :
- N-cinnamoyl-L-thréonine,
- N-(4-méthoxycinnamoyl)-L-thréonine,
- N-(3,4-diméthoxycinnamoyl)-L-thréonine,
- N-cafféoyl-L-thréonine.

L'invention a encore pour objet un procédé de préparation des composés répondant à la formule (Ib) ci-dessous : 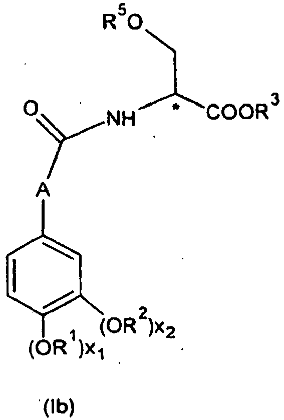 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L,
R⁵ représente un groupement choisi parmi : H, les alkyles en C₁-C₆, le phényle, le benzyle, les groupements -CO-R⁶ et R⁶ représente un groupement choisi parmi : les alkyles en C₁-C₆, le phényle, le benzyle,
à l'exclusion des composés suivants :
A représente -CH=CH-, x₁ = 1, x₂ = 1, et
   - R¹ = R² = CH₃CO-, R³ = CH₃, R⁵ = CH₃CO-, ou
   - R¹=R²=CH₃CO-, R³=H, R⁵=H, ou
   - R¹= R² = CH₃CO-, R³ = tBu, R⁵ = tBu.

De façon avantageuse, dans la formule (Ib) une ou plusieurs des conditions suivantes sont satisfaites :
A représente -CH=CH-,
x₁ est un entier choisi parmi 0 et 1,
x₂ est un entier choisi parmi 0 et 1,
R¹, R², indépendamment l'un de l'autre représentent un groupement choisi parmi : H, CH₃,
R³ représente H,
* représente la configuration optique L,
R⁵ représente H.

De façon préférée, le composé de formule (Ib) est choisi parmi la liste suivante :
- N-cinnamoyl-L-sérine,
- N-(4-méthoxycinnamoyl)-L-sérine,
- N-(3,4-diméthoxycinnamoyl)-L-sérine,

L'invention a encore pour objet un procédé de préparation des composés répondant à la formule (Ic) ci-dessous : 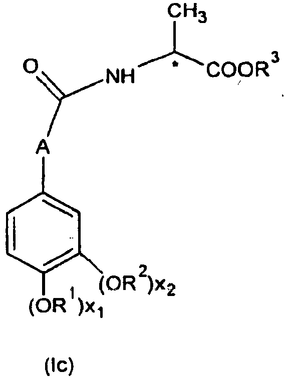 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L ou D,
à l'exclusion des cas où :
* représente la configuration optique L et
A représente -CH=CH-, et
-x₁ = x₂ = 0, R³ = tBu,

De façon avantageuse, dans la formule (Ic) une ou plusieurs des conditions suivantes sont satisfaites :
A représente -CH=CH-,
   - x₁ est un entier choisi parmi 0 et 1,
   - x₂ est un entier choisi parmi 0 et 1,
R¹, R², indépendamment l'un de l'autre représentent un groupement choisi parmi : H, CH₃,
R³ représente H.

De façon préférée, le composé de formule (Ic) est choisi parmi la liste suivante :
- N-cinnamoyl-L-alanine,
- N-(4-méthoxycinnamoyl)-L-alanine,
- N-(3,4-diméthoxycinnamoyl)-L-alanine,
- N-cafféoyl-L-alanine,
- N-cinnamoyl-D-alanine,
- N-(4-méthoxycinnamoyl)-D-alanine,
- N-(3,4-diméthoxycinnamoyl)-D-alanine,
- N-coumaroyl-D-alanine,
- N-cafféoyl-D-alanine.

Enfin, l'invention a aussi pour objet un procédé de préparation des composés répondant à la formule (Id) ci-dessous : 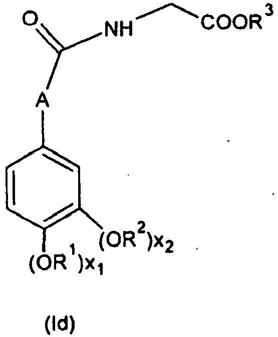 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
à l'exclusion des cas où :
A représente -CH=CH- et :
   - x₁= 1, x₂=0, R¹=CH₃, R³=H, ou
   - x₁ = 1, x₂ = 0, R¹ = CH₃, R³ = C₂H₅, ou
   - x₁ = x₂ = 0, R³=H.

De façon avantageuse, dans la formule (Id) une ou plusieurs des conditions suivantes sont satisfaites :
A représente -CH=CH-,
x₁ est un entier choisi parmi 0 et 1,
x₂ est un entier choisi parmi 0 et 1,
R¹, R², indépendamment l'un de l'autre représentent un groupement choisi parmi : H, CH₃,
R³ représente H.

De façon préférée, le composé de formule (Id) est le N-(3,4-diméthoxycinnamoyl) glycine.

Les composés obtenus selon le procédé de l'invention ont une activité antioxydante et antimicrobienne. Ils sont susceptibles d'agir sur l'interaction plaquettes-leucocytes et comme anti-inflammatoires. Ils peuvent être employés dans de nombreuses applications :
- En pharmacie, en association avec un support pharmaceutique, pour la préparation de compositions thérapeutiques destinées à la prévention ou au traitement des pathologies suivantes : cancer ; maladies inflammatoires ; maladies cardiovasculaires telles que l'athérosclérose, l'infarctus du myocarde, l'accident vasculaire cérébral, l'hypercholestérolémie ; maladies infectieuses telles que les infections dues à des virus par exemple.
- En cosmétique, en association avec un support cosmétique, comme conservateur, comme actif anti-âge, comme agent dépigmentant pour la peau et les phanères.
- En alimentaire également, les composés de l'invention peuvent être utilisés comme conservateurs dans de nombreuses applications.

### 1. PARTIE EXPERIMENTALE

### 1.1 Conditions de culture de la bactérie

### 1.1.1 Souche bactérienne

- Souche bactérienne utilisée : *Bacillus subtilis subsp. subtilis.*
- Morphologie et physiologie de la bactérie : Bâtonnets droits (0,5-2,5 x 1,2-10 microns) isolés ou en chaînes plus ou moins longues, à spore unique très résistante. La coloration de Gram est positive, surtout en début de croissance ; elle peut également être négative. Mobiles par flagelles péritriches. Aérobies ou anaérobies facultatifs. Catalase présente. Oxydase positive ou négative. Chimio-organotrophes, prototrophes à auxotrophes exigeant plusieurs facteurs de croissance. Le peptidoglycane de la paroi est du type acide *meso*-diamino-pimélique. Les phospholipides dominants sont la phosphatidyléthanolamine et le phosphatidylcérol. La formation de la spore est un processus multiphasique qui comprend sept étapes successives. La levée de la dormance implique trois processus séquentiels : l'activation, la germination et la croissance. De très nombreuses études génétiques ont été réalisées sur *B. subtilis* qui est l'espèce type du genre ; plus de 355 gènes ont été localisés sur la carte chromosomique. Grâce à l'extrême résistance de leur spore, les *Bacillus* sont ubiquistes. Leur habitat primaire est le sol où ils jouent un rôle important dans les cycles du carbone et de l'azote. Ils peuvent aussi contaminer les aliments.
- Numéros de collection : DSM N° 10, NCIB 3610, ATCC 6051 **(DSMZ,** Deutsche Sammlung von Mikroorganismen und Zellkulturen, Brunschweig, Germany; **NCIB,** National Center for Biotechnology Information. **ATCC,** The Global Bioresource center).
- Référence Bibliographique : Nakamura et al., Int. J. Syst. Bacteriol., 49:1211-1215,1999.

### 1.1.2 Milieux de culture

La synthèse des molécules s'effectue par culture de la bactérie. Des essais ont été faits avec un milieu dépourvu de fer, comparativement à une culture réalisée dans les mêmes conditions mais en présence de fer. Les milieux de culture utilisés sont les suivants :
Milieu 1. Il contient par litre : KH₂PO₄ 0,5 g, K₂HPO₄ 0,5 g, NaCl 0,4 g, NH₄Cl 0,4 g, extrait de levure (YE, de chez PANREAC (France), référence 403687.1210) 2 g. Le pH est ajusté à 7,0 avec une solution de KOH 10 M. Puis le milieu est autoclavé à 120°c pendant 20 min. En routine, des cultures de 25 mL sont réalisées dans des erlenmeyers de 50 mL.
Milieu 2. Celui-ci est identique au milieu 1 excepté l'extrait de levure qui est remplacé par le Yeast Nitrogen Basal (YNB, de chez DIFCO (France), référence 233520) qui ne contient pas d'acides aminés ni de sulfate d'ammonium.

### 1.1.3 Préparation des précurseurs (II) et (III) et de l'extrait de levure

Les composés (II) ont été préparés en anaérobiose (Hungate, 1969. In Methods in Microbiology, pp. 117-132. Edited by Norris, J. R. & Ribbons, D. W. London: Academic Press), stérilisés par filtration (filtre Millipore, porosité 0,22 µm), et conservés en flacons pénicilline sous N₂ à température ambiante et à l'abri de la lumière (flacons couverts avec du papier d'aluminium).

Les solutions mères de composés (II) acides sont préparées à une concentration de 250 mM, et ont été neutralisés à pH 7 avec la soude (0,4 g de pastilles pour 25 mL de solution mère). Les composés (II) suivants ont été testés : acide cinnamique, acide 4-méthoxycinnamique, acide 3,4-diméthoxycinnamique.

Les solutions mères de composés (III) sont préparées dans les mêmes conditions que les composés (II) mais à une concentration de 50 mM. Les composés (III) suivants ont été testés : glycine, L-alanine, D-alanine, L-sérine, D-sérine, L-thréonine, D-thréonine, L-valine, D-valine, L-leucine, D-leucine, L-isoleucine, D-isoleucine, L-phénylalanine, D-phénylalanine, L-tyrosine, D-tyrosine, acide L-aspartique, acide D-aspartique, acide L-glutamique, acide D-glutamique, L-asparagine, D-asparagine, L-glutamine, D-glutamine, L-cystéine, D-cystéine, L-histidine, D-histidine, L-lysine, D-lysine, L-méthionine, D-méthionine, L-proline, D-proline, L-tryptophane, D-tryptophane. Les composés de formule (III) ont été testés soit isolément en utilisant un milieu de culture dépourvu d'acides aminés (c'est-à-dire avec YNB comme extrait de levure), soit en mélange.

Les solutions mères d'extraits de levure YE [25 % (p/v)] (yeast extract de chez FLUTA, Référence 70161) et YNB (yeast nitrogen base (YNB) de chez DIFCO également, Référence 233520) sont préparées et stérilisées à l'autoclave pendant 20 min à 121°C.

### 1.1.4 Conditions de culture pour la production usuelle d'un seul métabolite

La souche est préalablement décongelée, puis ensemencée dans le milieu 2 contenant 1 mM de substrat (II) (préculture). Après 24 h à 30°c dans des incubateurs INFORS, à une agitation de 150 tours/min, les cultures sont inoculées avec 10 % (v/v) de la préculture dans le milieu 2 contenant 1 mM de substrat aromatique (II) et 2 mM d'acide aminé (III). Les cultures sont enfin mises en agitation dans les mêmes conditions pendant 48 h.

Volume(s) de culture : les expériences ont été réalisées à partir de cultures de 25 ml ou de 1 litre, dans des erlenmeyers de 50 mL ou des bouteilles de 2 L cotonnées, respectivement.

### 1.2 Extraction, purification et identification des composés

### 1.2.1 Extraction, purification

Après centrifugation de la culture de 25 mL, le surnageant est acidifié par quelques gouttes d'acide acétique glacial ou formique jusqu'à un pH final de 2, puis extrait 3 fois par de l'acétate d'éthyle dans une ampoule à décanter. Après évaporation à sec de la phase organique, deux orientations sont possibles :
- Premièrement, il est possible de garder cette fraction sèche qui contient environ 90-95% du composé désiré. Un contrôle par HPLC est réalisé dans ce cas en reprenant l'extrait sec dans du méthanol et en injectant 10 µl dans un système HPLC phase inverse.
- Deuxièmement, l'extrait sec est repris par de l'eau acidifiée (1% HCOOH), et est déposé sur une cartouche SePack C₁₈ (WATERS, France) qui est rincée par 10-15 ml d'eau acidifiée (1% HCOOH). La molécule est ensuite éluée par du méthanol pur, l'éluat est évaporé à l'aide d'un évaporateur rotatif sous vide BUCHI et le solide obtenu placé dans un pilulier. Les composés peuvent aisément être conservés à température ambiante ou à 4°C sous forme solide, à -20°C sous forme liquide dans du méthanol.

Dans le cas de cultures de 1 litre, le résidu sec obtenu après l'étape d'extraction à l'acétate d'éthyle est repris par de l'eau acidifiée à 1% d'acide formique puis déposé sur une colonne LICHROPREP RP18 (Merck, Ref. 1.09303.0100, volume de la phase 7 ml). Celle-ci est ensuite rincée par 3 volumes d'eau acidifiée (1% HCOOH), et les composés sont finalement élués par du méthanol pur ; après évaporation, le solide est récupéré dans un flacon et pesé.

### 1.2.2 Contrôle de routine par HPLC

Après avoir prélevé 1 ml de culture bactérienne et après centrifugation (8000 g, 5 min), 400 µL de surnageant sont acidifiés par 20 µL d'acide acétique glacial puis centrifugés à nouveau (précipitation des protéines extracellulaires). Enfin, 10 à 20 µL du surnageant obtenu est injecté dans le système HPLC. L'analyse et le contrôle peuvent également s'effectuer à partir de la molécule pure obtenue ci-dessus.

Les analyses HPLC sont effectuées sur une chaîne WATERS équipée d'un dégazeur à membrane, d'un injecteur RHEODYNE 7725i (La Jola, USA), d'une pompe binaire 1525, d'un four thermostaté et d'un détecteur à barrette de diode 2996. L'appareil est piloté par le logiciel Millenium 32 version 4.0. La séparation est assurée par une colonne C₁₈ SYMETRY (150 x 4.6 mm, porosité 5 µm, WATERS). La phase mobile, entraînée à un débit de 0,8 mL/min est composée de deux solvants : l'acétonitrile (A) et l'eau distillée acidifiée à 1 % d'acide acétique (B). La durée totale de l'élution est de 55 min. Le gradient utilisé comporte trois étapes :
Etape 1 : de 5 à 20 % de A dans B pendant 30 min ;
Etape 2, de 20 à 100 % de A dans B pendant 20 min ;
Etape 3, retour à 5 % de A dans B en 5 min.

L'identification des composés s'effectue, d'une part, par la détermination des temps de rétention et d'autre part grâce aux spectres UV/VIS déterminé pour chaque composé. Des molécules pures ont été préalablement préparées et servent de standards pour l'HPLC (temps de rétention) et pour la spectroscopie (UV/VIS, LC-MS, GC-MS).

### 1.2.3 Identification par LC-MS (couplage HPLC-spectrométrie de masse)

L'HPLC est un chromatographe HP 1100 (AGILENT TECHNOLOGIES, France). La séparation est assurée par une colonne C₁₈ SYMMETRY (Waters, France), de dimension 4,6 x 250 mm, et de porosité de 5 µm. Le système est équipé d'un injecteur automatique, d'un dégazeur à membrane, d'un four et d'un détecteur mono-longueurs d'onde variables. Le spectromètre de masse utilisé est un appareil de chez APPLIED BIOSYSTEMS (France), modèle SCIEX Api150EX qui utilise le mode d'ionisation par électro-spray. L'élution, l'acquisition et le traitement des données sont assurés respectivement par les logiciels CHEMSTATION et MASSCHROM version 1.1.

Conditions opératoires : Débit 0,3 ml/min ; température du four 30°C ; longueur d'onde (λmax) fixée à 278 nm dans le cas où le composé (II) n'est pas ramifié en position 3 ou 4 du cycle (x₁ = x₂ = 0) ; longueur d'onde (λmax) fixée à 310 nm dans les autres cas. La phase mobile est formée par un gradient de deux solvants : H₂O milli-Q acidifiée à l'acide formique à 1% (solvant A) et acétonitrile pur (solvant B). Le gradient utilisé s'effectue comme suit : étape 1, de 5 à 30% de B pendant 60 min ; étape 2 : de 30 à 100% de B pendant 30 min (durée totale 90 min).

Les conditions de masse sont les suivantes : valeurs de *m*/*z* de 30 à 3000 uma (unité de masse atomique), temps d'acquisition de 15 ms/scan, voltage du cône 20 ou 60 Volts, voltage du capillaire 4500 Volts, en mode positif et négatif, pression du nébulisateur de 45 psi.

### 1.2.4 Identification par GC-MS (couplage chromatographie en phase gazeuse-spectrométrie de masse)

Préalablement, sur le composé purifié solide ou un extrait sec contenant un mélange de composés, on procède à une réaction de dérivation par le BSTFA+TMCS ou d'estérification méthylique.

### Dérivation

Les réactions ont lieu dans un tube CHROMACOL de 2 ml scellé par un bouchon et un septum hermétique. Deux types de dérivation ont été réalisés dans ce travail :
- Trimethylsilylation avec le BSTFA contenant un catalyseur : Cette technique est utilisée pour les composés aromatiques lorsque la réaction de dérivation est plus difficile, le catalyseur étant ici le TMCS (triméthylchlorosilane). A 0,3-1 mg d'échantillon sont ajoutés 100 µL de pyridine et 100 µL de réactif BSTFA contenant 1% de TMCS (triméthylchlorosilane) (de chez SIGMA-ALDRICH, référence T6381). La solution est mise à l'étuve pendant 15 à 20 min à 60°C. Elle est ensuite évaporée sous azote et le résidu sec repris dans le méthanol ou l'acétate d'éthyle (100 à 500 µL) pour analyse en GC-MS.
- Estérification méthylique : Cette méthode a été mise au point spécifiquement pour les composés N-cinnamoyl amides d'acides aminés. 100 µL de PFPA (anhydride pentafluoropropionique, SIGMA-ALDRICH N° 394904) sont ajoutés à environ 0,3-1 mg d'échantillon dissout dans 100 µL de méthanol. La solution obtenue est ensuite mise à l'étuve pendant 30 min à 60°C. Le PFPA est évaporé sous azote et le résidu sec est repris dans de l'acétate d'éthyle (100 à 500 µL) pour analyse en GC-MS.

### Analysés

Les analyses ont été menées avec un appareil GC-MS (AGILENT TECHNOLOGIES) composé d'un chromatographe 6890N GC system, d'un spectromètre de masse 5973 Mass Selectiv Detector équipé d'une source d'ion à impact électronique et d'un analyseur de type quadrupôle, d'un logiciel d'acquisition MSD-CHEMSTATION ainsi que des banques (NIST, WILEY). Les composés sont séparés à l'aide d'une colonne capillaire DB-1MS (30 m x 0,25 mm, de chez JW Scientific) et dont les limites de température sont comprises entre -60°C et 350°C. La phase mobile est l'hélium. La pression dans la colonne est de 10,5 psi et le débit de 1 mL/min. La température de l'injecteur (Inlet) est de 280°C. La programmation du gradient de température est la suivante : 1 min à 100°C, puis croissant de 100 à 260°C à raison de 4°/min, puis 10 min à 260°C. La durée totale de l'élution est de 51 min. Les composés aromatiques sont préalablement dérivés pour les rendre plus volatils.

### II. RESULTATS

### 2.1 Figure

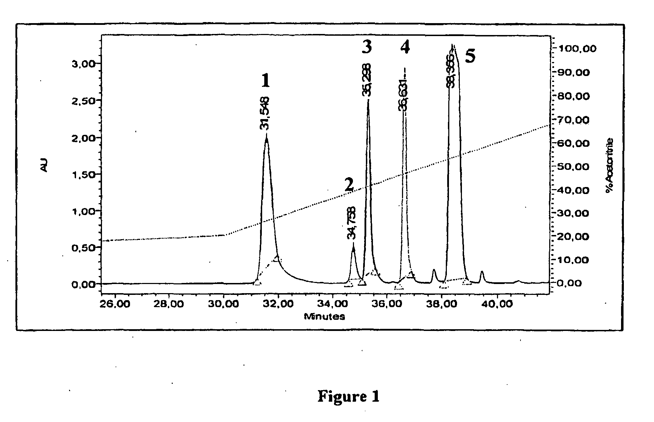

**Figure 1****.** Chromatogramme HPLC des composés N-cinnamoyl amides obtenus à partir d'une culture réalisée sur extrait de levure. Culture de 25 mL réalisée dans le milieu 1. 1, acide 4-méthoxy-N-cinnamoyl-L-sérine ; 2, acide 4-méthoxy-N-cinnamoylglycine ; 3, acide 4-méthoxy-N-cinnamoyl-L-thréonine ; 4, acide 4-méthoxy-N-cinnamoyl-(D,L)-alanine ; 5, acide 4-méthoxycinnamique, substrat résiduel dans la culture.

### 2.2 Molécules synthétisées à partir du milieu 1

Le milieu 1 contient de l'extrait de levure ; il n'y a pas d'apport supplémentaire d'acides aminés dans ce milieu, l'extrait de levure en contient déjà. Lorsque la bactérie croit dans ce milieu en présence d'acide cinnamique, ou d'acide 4-méthoxycinnamique, ou encore d'acide 3,4-diméthoxycinnamique, elle fabrique des composés N-cinnamoyl amides d'acides aminés uniquement lorsque ce milieu est dépourvu de fer, c'est-à-dire dans les conditions décrites dans la partie expérimentale. En effet, il n'y a pas de synthèse de molécules du type N-cinnamoyl amides en présence de fer (0,3 mg/L additionnés dans le milieu), cela après analyse par HPLC et contrôle des structures chimiques par LC-MS et GC-MS.

L'extraction du surnageant acidifié par de l'acétate d'éthyle et l'analyse des molécules en LC-MS nous ont permis de mettre clairement en évidence 5 molécules (**Figure 1**). Ainsi, la L-sérine, la L-thréonine, la L- ou la D- alanine apportées par le milieu de culture sont condensées avec le précurseur (II) pour former les acides 4-méthoxy-N-cinnamoyl-L-sérine, 4-méthoxy-N-cinnamoyl-L-thréonine, 4-méthoxy-N-cinnamoyl-L-alanine et 4-méthoxy-N-cinnamoyl-D-alanine. L'enzyme ne fait aucune différence entre les deux formes L et D de l'alanine. On a également obtenu en petite quantité l'acide 4-méthoxy-N-cinnamoyl-glycine.

En prenant l'acide cinnamique ou l'acide 3,4-diméthoxycinnamique pour le composé (II), on obtient de la même manière pour chaque substrat les molécules correspondantes (dérivés amides de L-sérine, L-thréonine, L-alanine et D-alanine, glycine).

Après hydrolyse de la liaison amide par reflux en présence d'H₂SO₄ pendant 6 heures, extraction de la solution à l'acétate d'éthyle et évaporation à sec de la phase aqueuse, l'analyse GC-MS après dérivation au BSTFA révèle la présence de glycine, de sérine, de thréonine et d'alanine, permettant ainsi de confirmer les structures des molécules.

**Conclusions** : On observe la formation des molécules 1, acide 4-méthoxy-N-cinnamoylsérine ; 2, acide 4-méthoxy-N-cinnamoylglycine ; 3, acide 4-méthoxy-N-cinnamoylthréonine ; 4, acide 4-méthoxy-N-cinnamoylalanine. La sérine, la thréonine, l'alanine et probablement la glycine sont présents dans l'extrait de levure YE. Une ou plusieurs enzymes de la bactérie font réagir ces acides aminés sur le précurseur (II) (ici l'acide 4-méthoxycinnamique), les acides aminés étant apportés par l'extrait de levure.

### 2.3 Cultures réalisées avec le milieu 2 pour la production orientée d'une seule molécule

Un milieu de culture (milieu 2) dépourvu d'acides aminés permet d'obtenir une seule molécule à la fois, cela par l'addition de l'acide aminé (composé (III) dans ce même milieu). De cette manière, cela a permis de connaître la stéréosélectivité de la réaction vis-à-vis de l'acide aminé (D ou L). Quatre cultures ont été réalisées en présence de YNB (extrait de levure ne contenant pas d'acides aminés, ni de sulfate d'ammonium), en y ajoutant un seul acide aminé par culture à une concentration de 2 mM, et en présence d'acide 4-méthoxycinnamique à 1 mM. Après 72 heures de culture, l'analyse par HPLC montre que, comparativement à la Figure 1, le substrat aromatique est totalement transformé. De plus, dans ces conditions, un seul composé N-cinnamoyl amide est produit. Les caractéristiques spectrales et chimiques de N-cinnamoylamides obtenus à partir de l'acide 4-méthoxycinnamique sont résumées en exemple dans le **Tableau 1** ci-dessous.

**Tableau 1. Caractéristiques et taux de production des composés N-cinnamoyl amides d'acides aminés obtenus par culture de la bactérie dans le milieu 2. Composé (II), acide 4-méthoxycinnamique 1 mM ; composé (III), l'acide aminé mentionné ici à 2 mM.**

| **Composé** | **RT (min)¹** | **λmax (nm)²** | **Masse de l'ester méthylique³** | **Production⁴ (mg/L)** |
|---|---|---|---|---|
| Acide 4-méthoxycinnamique | 38,35 | 307 | 220 | 0 |
| 4-méthoxy-N-cinnamoyl-L-sérine | 31.96 | 307 | 279 | 198 |
| 4-méthoxy-N-cinnamoyl-D-sérine | - | - | 279 | 0 |
| 4-méthoxy-N-cinnamoyl-L-thréonine | 35.26 | 307 | 293 | 176 |
| 4-méthoxy-N-cinnamoyl-D-thréonine | - | - | 293 | 0 |
| 4-méthoxy-N-cinnamoyl-L-alanine | 36,43 | 307 | 263 | 191 |
| 4-méthoxy-N-cinnamoyl-D-alanine | 36,44 | 307 | 263 | 173 |
| 4-méthoxy-N-cinnamoylglycine | 34.76 | 307 | 249 | ND |

1. RT, temps de rétention en HPLC déterminé sur une chaîne (cf. partie expérimentale). 2. Longueur d'onde maximale des molécules déterminée à partir de l'analyse HPLC. 3. Masse de l'ester méthylique calculée. Seuls, les dérivés de la glycine et de l'alanine montrent un ion moléculaire équivalent à cette valeur en GC-MS ; pour les dérivés de la sérine et de la thréonine, ils forment en GC-MS un alcène correspondant à la réaction de déshydratation de la fonction alcool primaire et secondaire, respectivement. 4. Les quantités ont été déterminées pour 1 litre de culture après 72 h, après extraction du surnageant à l'acétate d'éthyle et purification sur support chromatographique C₁₈ (cf. partie expérimentale). ND, non déterminé.

### 2.4 Stéréosélectivité de l'enzyme vis-à-vis de l'acide aminé (D ou L)

Les expériences montrent que l'enzyme bactérienne lie la L-sérine et la L-thréonine (et non la forme D) sur le substrat aromatique, alors qu'elle ne différencie pas la L- ou la D-alanine. La glycine, quant à elle, ne possède pas de carbone asymétrique (cf. **Tableau 1** à titre d'exemple).

### 2.5 Relations structure aromatique / activité

L'enzyme n'agit que sur les composés cinnamiques non hydroxylés. Nous avons également testés suivant le même protocole plusieurs substrats aromatiques : les acides p-hydroxybenzoiques, protocatéchuique, cinnamique, p-coumarique, férulique, 4-méthoxycinnamique, 3,4-diméthoxycinnamique, 3,4,5-triméthoxycinnamique, et catéchol. Seuls, les acides cinnamique, 4-méthoxycinnamique, et 3,4-diméthoxycinnamique, ont été transformés en dérivés N-cinnamoylamides. Ainsi, deux remarques sont à évoquer ici :
(1) La synthèse s'effectue avec des composés de structure équivalente à la formule (II).
(2) Lorsqu'un seul groupement -OH est présent sur le cycle aromatique (position 3, 4 ou 5), la synthèse ne se fait plus.

### 2.6 Taux de production à partir d'1 litre de culture (milieu 2)

Un litre de culture (milieu 2) contenant 2 mM d'un seul acide aminé (choisi parmi : L-alanine, D-alanine, L-sérine, L-thréonine) et 1 mM d'acide 4-méthoxycinnamique (composé (II)), est mis à incuber à 30°C pendant 72 h (agitation 150 tours/min). Après extraction à l'acétate d'éthyle, purification sur support C₁₈ comme décrit ci-dessus, et contrôle de la structure par HPLC, LC-MS et GC-MS, on obtient les quantités suivantes (cf. **Tableau 1**) :

| | |
|---|---|
| 4-méthoxy N-cinnamoyl-L-serine | : 198 mg pour 1 litre |
| 4-méthoxy N-cinnamoyl-L-threonine | : 176 mg pour 1 litre |
| 4-méthoxy N-cinnamoyl-L-alanine | : 191 mg pour 1 litre |
| 4-méthoxy N-cinnarnoyl-D-alanine | : 173 mg pour 1 litre |

## Revendications

1. Procédé de préparation de molécules répondant à la formule (I) ci-dessous : 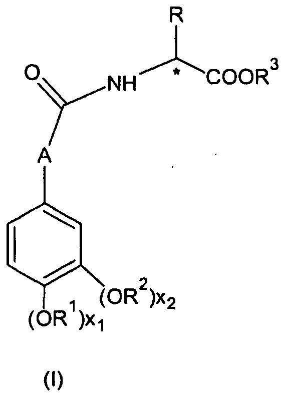 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre, représentent un groupement choisi parmi : H, les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L ou D de l'acide aminé -NH-CHR-COO-, * peut représenter l'une ou l'autre des configurations suivant le choix de R,
R représente une chaîne latérale d'acide aminé choisie parmi : -H (glycine), -CH₃ (L-alanine, D-alanine), -CH₂OH (L-sérine), -CHOH-CH₃ (L-thréonine), - CH₂OR⁵ (L-sérine protégée sur sa fonction hydroxyle), -CHOR⁵-CH₃ (L-thréonine protégée sur sa fonction hydroxyle) et R⁵ représente un groupement choisi parmi : H, les alkyles en C₁-C₆, le phényle, le benzyle, les groupements -CO-R⁶ et R⁶ représente un groupement choisi parmi : les alkyles en C₁-C₆, le phényle, le benzyle,
ce procédé comportant au moins une étape de culture d'au moins une bactérie choisie parmi les Bacillaceae, en présence d'au moins deux substrats sélectionnés parmi ceux répondant aux formules (II) et (III) ci-dessous : 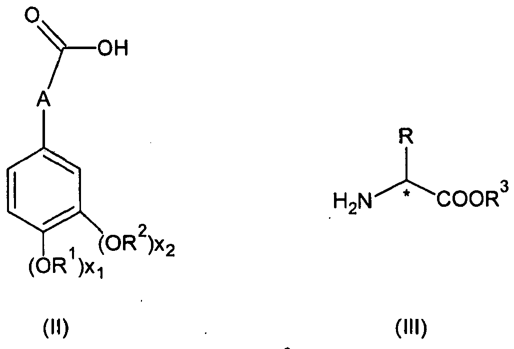
dans lesquelles A, x₁, x₂, R³,* et R ont la même définition que dans la formule (I),
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle.

2. Procédé selon la revendication 1 dans lequel la bactérie est choisie parmi celles appartenant au genre *Bacillus*, et avantageusement parmi : *Bacillus subtilis, Bacillus licheniformis, Bacillus thermoamylovorans, Bacillus* (ou *Geobacillus) stearothermophilus, Bacillus caldotenax.*

3. Procédé selon la revendication 2, dans lequel la bactérie est choisie parmi :
• *Bacillus subtilis* subsp. *Spizizenii* : DSM 15029
• *Bacillus licheniformis* : DSM 13, ATCC 14580, NCIB 9375
• *Bacillus thermoamylovorans* : DSM 13307
• *Bacillus* (ou *Geobacillus) stearothermophilus* : DSM 22, ATCC 12980, NCIB 8923
• *Bacillus caldotenax* : DSM 406

4. Procédé selon l'une quelconque des revendications 1 à 3, dans lequel dans la formule (I) une ou plusieurs des conditions ci-dessous sont satisfaites :
A représente un groupement -CH=CH- ,
R¹, R², indépendamment l'un de l'autre représentent un groupement choisi parmi : H, un groupement méthyle,
R³ représente H,
R représente une chaîne latérale d'acide aminé choisie parmi : -H (glycine), -CH₃ (L-alanine, D-alanine), -CH₂OH (L-sérine), -CHOH-CH₃ (L-thréonine),

5. Procédé selon l'une quelconque des revendications 1 à 4, dans lequel la culture de la bactérie est faite dans un milieu qui comporte des sels minéraux et un extrait de levure.

6. Procédé selon l'une quelconque des revendications 1 à 5, dans lequel la culture de la bactérie est faite dans un milieu qui comporte, outre le ou les acides aminés de formule (III), au moins un autre acide aminé choisi parmi : la lysine, la proline, la cystéine.

7. Procédé selon l'une quelconque des revendications 1 à 6, dans lequel le précurseur (II) est introduit dans le milieu de culture sous forme d'un extrait végétal.

8. Procédé selon l'une quelconque des revendications 1 à 7, qui comporte avantageusement les étapes suivantes :
- centrifugation,
- récupération du surnageant,
- purification du produit (I).

9. Utilisation du procédé selon l'une quelconque des revendications 1 à 8 pour la préparation d'une molécule choisie parmi :
- N-cinnamoyl-glycine,
- N-cinnamoyl-L-alanine,
- N-cinnamoyl-D-alanine,
- N-cinnamoyl-L-sérine,
- N-cinnamoyl-L-thréonine,
- N-(4-méthoxycinnamoyl)-glycine,
- N-(4-méthoxycinnamoyl)-L-alanine,
- N-(4-méthoxycinnamoyl)-D-alanine,
- N-(4-méthoxycinnamoyl)-L-sérine,
- N-(4-méthoxycinnamoyl)-L-thréonine
- N-(3,4-diméthoxycinnamoyl)-glycine,
- N-(3,4-diméthoxycinnamoyl)-L-alanine,
- N-(3,4-diméthoxycinnamoyl)-D-alanine,
- N-(3,4-diméthoxycinnamoyl)-L-sérine,
- N-(3,4-diméthoxycinnamoyl)-L-thréonine,
- N-coumaroyl-L-thréonine,
- N-cafféoyl-L-thréonine,
- N-coumaroyl-L-sérine
- N-cafféoyl-L-sérine
- N-coumaroyl-L-alanine,
- N-cafféoyl-L-alanine,
- N-coumaroyl-D-alanine,
- N-cafféoyl-D-alanine,
- N-coumaroyl-glycine,
- N-cafféoyl-glycine.

10. Procédé selon l'une des revendications 1 à 9 pour la préparation d'un composé répondant à la formule (Ia) ci-dessous : 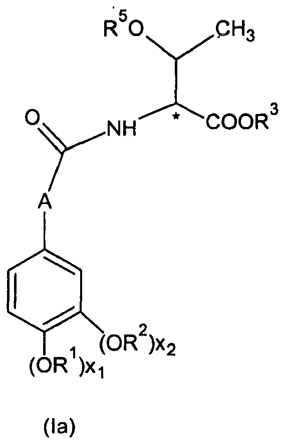 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L,
R⁵ représente un groupement choisi parmi : H, les alkyles en C₁-C₆, le phényle, le benzyle, les groupements -CO-R⁶ et R⁶ représente un groupement choisi parmi : les alkyles en C₁-C₆, le phényle, le benzyle.

11. Procédé selon l'une des revendications 1 à 9 pour la préparation d'un composé répondant à la formule (Ib) ci-dessous : 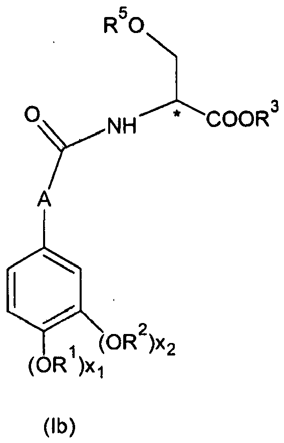 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L,
R⁵ représente un groupement choisi parmi : H, les alkyles en C₁-C₆, le phényle, le benzyle, les groupements -CO-R⁶ et R⁶ représente un groupement choisi parmi : les alkyles en C₁-C₆, le phényle, le benzyle,
à l'exclusion des composés suivants où :
A représente -CH = CH-, x₁ = 1, x₂ = 1, et
- R¹ = R² = CH₃CO-, R³ = CH₃, R⁵ = CH₃CO-, ou
- R¹ = R² = CH₃CO-, R³= H, R⁵= H, ou
- R¹ = R² = CH₃CO-, R³ = tBu, R⁵ = tBu

12. Procédé selon l'une des revendications 1 à 9 pour la préparation d'un composé répondant à la formule (Ic) ci-dessous : 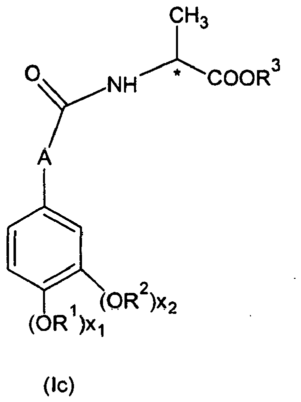 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
* représente la configuration optique L ou D,
à l'exclusion des cas où :
* représente la configuration optique L et
A représente -CH=CH-, et
- x₁ = x₂ = 0, R³ = tBu.

13. Procédé selon l'une des revendications 1 à 9 pour la préparation d'un composé répondant à la formule (Id) ci-dessous : 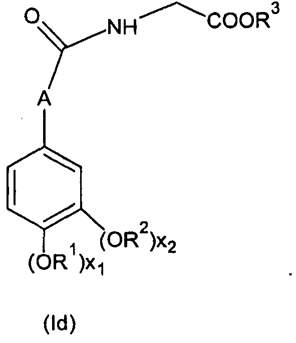 dans laquelle :
A représente un groupement choisi parmi -CH=CH- et -CH₂-CH₂-,
x₁ est un entier choisi parmi 0 et 1,
x₂ est un entier choisi parmi 0 et 1,
R¹, R², indépendamment l'un de l'autre représentent un groupement choisi parmi : les alkyles en C₁-C₆, le phényle, le benzyle, les groupements -CO-R⁴ et R⁴ représente un groupement choisi parmi : les alkyles en C₁-C₆, le phényle, le benzyle,
R³ représente un groupement choisi parmi : H, les alkyles en C₁-C₆, le phényle, le benzyle, une chaîne peptidique d'acides aminés comprenant de 1 à 30 acides aminés,
à l'exclusion des cas où :
A représente -CH=CH- et :
- x₁=1, x₂=0, R¹=CH₃, R³=H
- x₁=1, x₂=0, R¹=CH₃, R³=C₂H₅
- x₁=x₂=0, R³=H.

## Patentansprüche

1. Verfahren zur Herstellung von Molekülen, die der folgenden Formel (I) entsprechen: 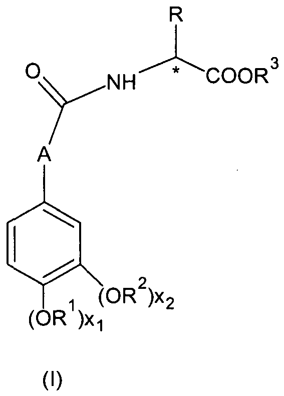 in welcher:
A eine Gruppe darstellt, die ausgewählt ist aus -CH=CH- und -CH₂-CH₂-,
x₁ eine ganze Zahl ist, ausgewählt aus 0 und 1,
x₂ eine ganze Zahl ist, ausgewählt aus 0 und 1,
R¹, R², unabhängig voneinander eine Gruppe darstellen, die ausgewählt ist aus: H, C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist,
R³ eine Gruppe darstellt, die ausgewählt ist aus: H, C₁-C₆-Alkyl, Phenyl, Benzyl, einer Peptidkette von Aminosäuren, die zwischen 1 und 30 Aminosäuren enthält,
* die optische Konfiguration L oder D der Aminosäure -NH-CHR-COO- darstellt, wobei * je nach der Auswahl von R die eine oder die andere Konfiguration darstellen kann,
R eine Aminosäureseitenkette darstellt, die ausgewählt ist aus: -H (Glycin), -CH₃ (L-Alanin, D-Alanin), -CH₂OH (L-Serin), -CHOH-CH₃ (L-Threonin), -CH₂OR⁵ (auf seiner Hydroxylfunktion geschütztes L-Serin), -CHOR⁵-CH₃ (auf seiner Hydroxylfunktion geschütztes L-Threonin), wobei R⁵ eine Gruppe darstellt, die ausgewählt ist aus: H, C₁-C₆-Alkyl, Phenyl, Benzyl, den Gruppen -CO-R⁶, wobei R⁶ eine Gruppe darstellt, die ausgewählt ist aus: H, C₁-C₆-Alkyl, Phenyl, Benzyl,
wobei das Verfahren wenigstens einen Schritt des Kultivierens mindestens eines Bakteriums aufweist, das aus den Bacillaceae ausgewählt ist, in Gegenwart von mindestens zwei Substraten, die aus denjenigen Substraten ausgewählt sind, die den folgenden Formeln (II) und (III) entsprechen: 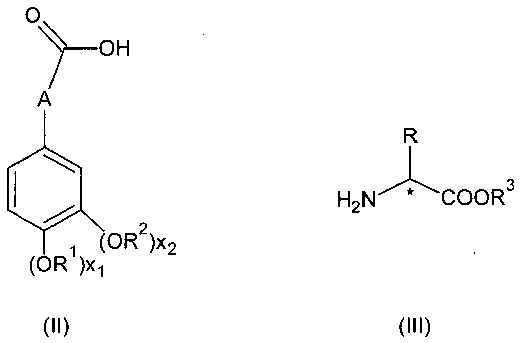
worin A, x₁, x₂, R³,* und R die gleiche Bedeutung wie in Formel (I) haben,
R¹, R² unabhängig voneinander eine Gruppe darstellen, die ausgewählt ist aus: C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist.

2. Verfahren nach Anspruch 1, in welchem das Bakterium aus den zur Gattung Bacillus gehörenden Bakterien ausgewählt ist, und vorzugsweise aus: *Bacillus subtilis, Bacillus licheniformis, Bacillus thermoamylovorans, Bacillus* (oder *Geobacillus*) *stearothermophilus, Bacillus caldotenax*.

3. Verfahren nach Anspruch 2, in welchem das Bakterium ausgewählt ist aus
• *Bacillus subtilis* subsp. *Spizizenii*: DSM 15029
• *Bacillus licheniformis* : DSM 13, ATCC 14580, NCIB 9375
• *Bacillus thermoamylovorans* : DSM 13307
• *Bacillus* (oder *Geobacillus*) *stearothermophilus* : DSM 22, ATCC 12980, NCIB 8923
• *Bacillus caldotenax* : DSM 406

4. Verfahren nach einem der Ansprüche 1 bis 3, in welchem in Formel (I) eine oder mehrere der folgenden Bedingungen erfüllt sind:
A bedeutet eine Gruppe -CH=CH-,
R¹, R² bedeuten unabhängig voneinander eine Gruppe, die aus H und einer Methylgruppe ausgewählt ist,
R³ bedeutet H,
R bedeutet eine Aminosäureseitenkette, die ausgewählt ist aus -H (Glycin), -CH₃ (L-Alanin, D-Alanin), -CH₂OH (L-Serin), -CHOH-CH₃ (L-Threonin).

5. Verfahren nach einem der Ansprüche 1 bis 4, bei dem die Bakterienkultur in einem Medium erfolgt, das anorganische Salze und einen Hefeextrakt enthält.

6. Verfahren nach einem der Ansprüche 1 bis 5, bei dem die Bakterienkultur in einem Medium erfolgt, das außer der oder den Aminosäuren der Formel (III) mindestens eine weitere Aminosäure enthält, die aus Lysin, Prolin, Cystein ausgewählt ist.

7. Verfahren nach einem der Ansprüche 1 bis 6, in welchem der Vorläufer (II) dem Kulturmedium als Pflanzenextrakt zugeführt wird.

8. Verfahren nach einem der Ansprüche 1 bis 7, das vorzugsweise folgende Schritte aufweist:
- Zentrifugierung,
- Gewinnung des Überstands,
- Reinigung des Produkts (I).

9. Verwendung des Verfahrens nach einem der Ansprüche 1 bis 8 zur Herstellung eines Moleküls, das ausgewählt ist aus:
- N-Cinnamoyl-Glycin,
- N-Cinnamoyl-L-Alanin,
- N-Cinnamoyl-D-Alanin,
- N-Cinnamoyl-L-Serin,
- N-Cinnamoyl-L-Threonin,
- N-(4-Methoxycinnamoyl)-Glycin,
- N-(4-Methoxycinnamoyl)-L-Alanin,
- N-(4-Methoxycinnamoyl)-D-Alanin,
- N-(4-Methoxycinnamoyl)-L-Serin,
- N-(4-Methoxycinnamoyl)-L-Threonin
- N-(3,4-Dimethoxycinnamoyl)-Glycin,
- N-(3,4-Dimethoxycinnamoyl)-L-Alanin,
- N-(3,4-Dimethoxycinnamoyl)-D-Alanin,
- N-(3,4-Dimethoxycinnamoyl)-L-Serin,
- N-(3,4-Dimethoxycinnamoyl)-L-Threonin,
- N-Cumaroyl-L-Threonin,
- N-Caffeoyl-L-Threonin,
- N-Cumaroyl-L-Serin
- N-Caffeoyl-L-Serin
- N- Cumaroyl-L-Alanin,
- N-Caffeoyl-L-Alanin,
- N- Cumaroyl-D-Alanin,
- N-Caffeoyl-D-Alanin,
- N-Cumaroyl-Glycin,
- N-Caffeoyl-Glycin.

10. Verfahren nach einem der Ansprüche 1 bis 9 zur Herstellung einer der folgenden Formel (Ia) entsprechenden Verbindung: 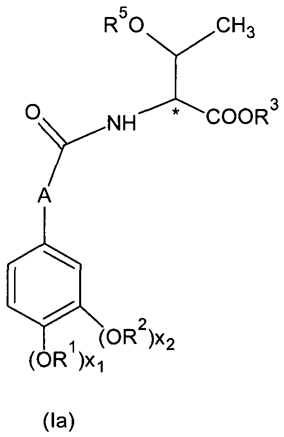 in welcher:
A eine Gruppe darstellt, die ausgewählt ist aus -CH=CH- und -CH₂-CH₂-,
x₁ eine ganze Zahl ist, ausgewählt aus 0 und 1,
x₂ eine ganze Zahl ist, ausgewählt aus 0 und 1, R¹, R², unabhängig voneinander eine Gruppe darstellen, die ausgewählt ist aus: H, C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist,
R³ eine Gruppe darstellt, die ausgewählt ist aus: H, C₁-C₆-Alkyl, Phenyl, Benzyl, einer Peptidkette von Aminosäuren, die zwischen 1 und 30 Aminosäuren enthält,
* die optische Konfiguration L bedeutet,
R⁵ eine Gruppe darstellt, die ausgewählt ist aus: H, C₁-C₆-Alkyl, Phenyl, Benzyl, den Gruppen -CO-R⁶, wobei R⁶ eine Gruppe darstellt, die ausgewählt ist aus H, C₁-C₆-Alkyl, Phenyl, Benzyl.

11. Verfahren nach einem der Ansprüche 1 bis 9 zur Herstellung einer der folgenden Formel (Ib) entsprechenden Verbindung: 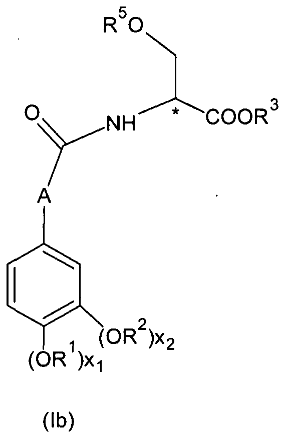 in welcher:
A eine Gruppe darstellt, die ausgewählt ist aus -CH=CH- und -CH₂-CH₂-,
x₁ eine ganze Zahl ist, ausgewählt aus 0 und 1,
x₂ eine ganze Zahl ist, ausgewählt aus 0 und 1,
R¹, R², unabhängig voneinander eine Gruppe darstellen, die ausgewählt ist aus: H, C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist,
R³ eine Gruppe darstellt, die ausgewählt ist aus: H, C₁-C₆-Alkyl, Phenyl, Benzyl, einer Peptidkette von Aminosäuren, die zwischen 1 und 30 Aminosäuren enthält,
* die optische Konfiguration L bedeutet,
R⁵ eine Gruppe darstellt, die ausgewählt ist aus: H, C₁-C₆-Alkyl, Phenyl, Benzyl, den Gruppen -CO-R⁶, wobei R⁶ eine Gruppe darstellt, die ausgewählt ist aus H, C₁-C₆-Alkyl, Phenyl, Benzyl,
mit Ausnahme der folgenden Verbindungen, in welchen:
A = -CH=CH-, X₁ = 1, X₂ = 1 darstellt, und
- R¹ = R² = CH₃CO-, R³ = CH₃, R⁵ = CH₃CO- ist, oder
- R¹ = R² = CH₃CO-, R³ = H, R⁵= H ist, oder
- R¹ = R² = CH₃CO-, R³ = tBu, R⁵ = tBu ist.

12. Verfahren nach einem der Ansprüche 1 bis 9 zur Herstellung einer der folgenden Formel (Ic) entsprechenden Verbindung: 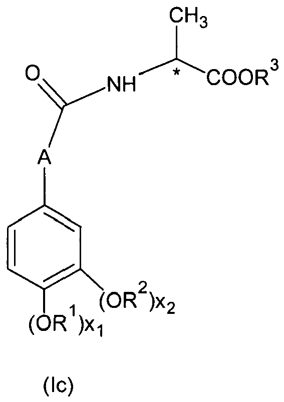 in welcher:
A eine Gruppe darstellt, die ausgewählt ist aus -CH=CH- und -CH₂-CH₂-,
x₁ eine ganze Zahl ist, ausgewählt aus 0 und 1,
x₂ eine ganze Zahl ist, ausgewählt aus 0 und 1,
R¹, R², unabhängig voneinander eine Gruppe darstellen, die ausgewählt ist aus: H, C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist,
R³ eine Gruppe darstellt, die ausgewählt ist aus: H, C₁-C₆-Alkyl, Phenyl, Benzyl, einer Peptidkette von Aminosäuren, die zwischen 1 und 30 Aminosäuren enthält,
* die optische Konfiguration L oder D bedeutet,
mit Ausnahme der Fälle, in welchen:
* die optische Konfiguration L bedeutet und
A -CH=CH- darstellt, und
- x₁ = x₂= 0, R³ = tBu ist.

13. Verfahren nach einem der Ansprüche 1 bis 9 zur Herstellung einer der folgenden Formel (Id) entsprechenden Verbindung: 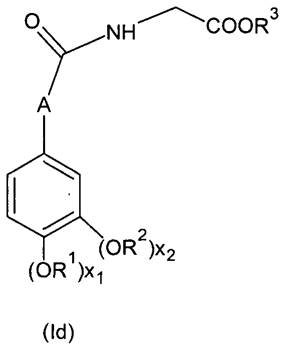 in welcher:
A eine Gruppe darstellt, die ausgewählt ist aus -CH=CH- und -CH₂-CH₂-,
x₁ eine ganze Zahl ist, ausgewählt aus 0 und 1,
x₂ eine ganze Zahl ist, ausgewählt aus 0 und 1,
R¹, R², unabhängig voneinander eine Gruppe darstellen, die ausgewählt ist aus: H, C₁-C₆-Alkyl, Phenyl, Benzyl, den Gruppen -CO-R⁴, wobei R⁴ eine Gruppe darstellt, die aus C₁-C₆-Alkyl, Phenyl, Benzyl ausgewählt ist,
R³ eine Gruppe darstellt, die ausgewählt ist aus: H, C₁-C₆-Alkyl, Phenyl, Benzyl, einer Peptidkette von Aminosäuren, die zwischen 1 und 30 Aminosäuren enthält,
mit Ausnahme der Fälle, in welchen:
A = -CH=CH- ist und:
- x₁=1, x₂=0, R¹=CH₃, R³=H ist,
- x₁=1, x₂=0, R¹=CH₃, R³=C₂H₅ ist,
- x₁ = x₂ = 0, R³ = H ist.

## Claims

1. Method of preparing molecules having the general formula (I) given below: 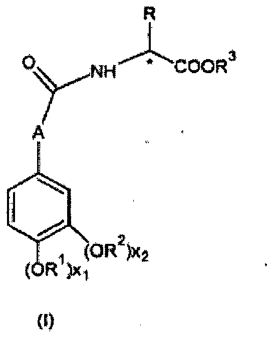 in which:
A is a group selected from -CH=CH- and -CH₂-CH₂-,
x₁ is an integer selected from 0 and 1,
x₂ is an integer selected from 0 and 1, R¹, R², independently of one another, are a group selected from: H, C₁-C₆ alkyls, phenyl and benzyl, and the groups -CO-R⁴ and R⁴ are a group selected from: C₁-C₆ alkyls, phenyl and benzyl,
R³ is a group selected from: H, C₁-C₆ alkyls, phenyl, benzyl, and a peptide chain of amino acids comprising from 1 to 30 amino acids,
* is the L or D optical configuration of the amino acid -NH-CHR-COO-, * being able to be either of these configurations depending on what R is selected to be,
R is an amino acid side chain selected from: -H (glycine), -CH₃ (L-alanine, D-alanine), -CH₂OH (L-serine), -CHOH-CH₃ (L-threonine), -CH₂OR⁵ (L-serine whose hydroxyl group is protected), -CHOR₅-CH₃ (L-threonine whose hydroxyl group is protected), and R⁵ is a group selected from: H, C₁-C₆ alkyls, phenyl, benzyl, and the groups -CO-R⁶ and R⁶ is a group selected from: C₁-C₆ alkyls, phenyl and benzyl,
said method comprising at least one step of culturing at least one bacterium selected from the Bacillaceae, in the presence of at least two substrates which are selected from those having the general formulas (II) and (III) given below: 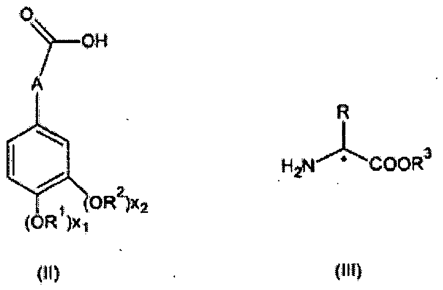
in which the definitions of A, x₁, x₂, R³, * and R are the same as in general formula (I),
R¹, R², independently of one another, are a group selected from: C₁-C₆ alkyls, phenyl, benzyl, and the groups -CO-R⁴ and R⁴ are a group selected from: C₁-C₆ alkyls, phenyl and benzyl.

2. Method according to claim 1, wherein the bacterium is selected from those belonging to the *Bacillus* genus, and advantageously from: *Bacillus subtilis, Bacillus licheniformis, Bacillus thermoamylovorans, Bacillus* (or *Geobacillus) stearothermophilus, Bacillus caldotenax.*

3. Method according to claim 2, wherein the bacterium is selected from:
• *Bacillus subtilis* subsp. *Spizizenii*: DSM 15029
• *Bacillus licheniformis*: DSM 13, ATTC 14580, NCIB 9375
• *Bacillus thermoamylovorans*: DSM 13307
• *Bacillus* (or *Geobacillus*) *stearothermophilus*: DSM 22, ATCC 12980, NCIB 8923,
• *Bacillus caldotenax*: DSM 406.

4. Method according to any one of claims 1 to 3, wherein one or more of the conditions given below are satisfied in general formula (I):
A is a group -CH=CH-,
R¹, R², independently of one another, are a group selected from: H and a methyl group,
R³ is H,
R is an amino acid side chain selected from: -H (glycine), -CH₃ (L-alanine, D-alanine), -CH₂OH (L-serine), -CHOH-CH₃ (L-threonine).

5. Method according to any one of claims 1 to 4,
wherein the culturing of the bacterium is carried out in a medium which comprises mineral salts and a yeast extract.

6. Method according to any one of claims 1 to 5,
wherein the culturing of the bacterium is carried out in a medium which comprises, in addition to the amino acid or acids of formula (III), at least one other amino acid selected from: lysine, proline, cysteine.

7. Method according to any one of claims 1 to 6,
wherein the precursor (II) is introduced into the culture medium in the form of a vegetable extract.

8. Method according to any one of claims 1 to 7, which advantageously comprises the following steps:
- centrifuging,
- recovery of the supernatant,
- purification of the product (I).

9. Use of the method according to any one of claims 1 to 8 for preparing a molecule selected from:
- N-cinnamoylglycine
- N-cinnamoyl-L-alanine
- N-cinnamoyl-D-alanine
- N-cinnamoyl-L-serine
- N-cinnamoyl-L-threonine
- N-(4-methoxycinnamoyl)glycine
- N-(4-methoxycinnamoyl)-L-alanine
- N-(4-methoxycinnamoyl)-D-alanine
- N-(4-methoxycinnamoyl)-L-serine
- N-(4-methoxycinnamoyl)-L-threonine
- N-(3,4-dimethoxycinnamoyl)glycine
- N-(3,4-dimethoxycinnamoyl)-L-alanine
- N-(3,4-dimethoxycinnamoyl)-D-alanine
- N-(3,4-dimethoxycinnamoyl)-L-serine
- N-(3,4-dimethoxycinnamoyl)-L-threonine
- N-coumaroyl-L-threonine
- N-caffeoyl-L-threonine
- N-coumaroyl-L-serine
- N-caffeoyl-L-serine
- N-coumaroyl-L-alanine
- N-caffeoyl-L-alanine
- N-coumaroyl-D-alanine
- N-caffeoyl-D-alanine
- N-coumaroylglycine
- N-caffeoylglycine.

10. Method according to any one of claims 1 to 9 for preparing a compound having the formula (Ia) given below: 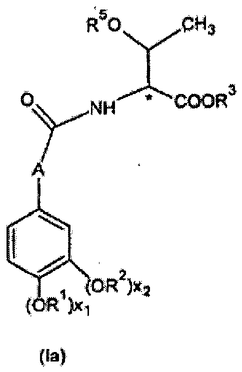 in which:
A is a group selected from -CH=CH- and -CH₂-CH₂-,
x₁ is an integer selected from 0 and 1,
x₂ is an integer selected from 0 and 1,
R¹, R², independently of one another, are a group selected from: C₁-C₆ alkyls, phenyl, benzyl, the groups -CO-R⁴ and R⁴ is a group selected from: C₁-C₆ alkyls, phenyl, benzyl,
R³ is a group selected from: H, C₁-C₆ alkyls, phenyl, benzyl, a peptide chain of amino acids comprising from 1 to 30 amino acids,
* is the L optical configuration,
R⁵ is a group selected from: H, C₁-C₆ alkyls, phenyl, benzyl, the groups -CO-R⁶ and R⁶ is a group selected from: C₁-C₆ alkyls, phenyl, benzyl.

11. Method according to any one of claims 1 to 9 for preparing a compound having the formula (Ib) given below: 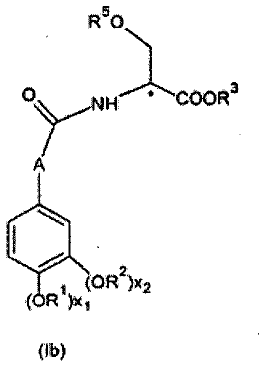 in which:
A is a group selected from -CH=CH- and -CH₂-CH₂-,
x₁ is an integer selected from 0 and 1,
x₂ is an integer selected from 0 and 1,
R¹, R², independently of one another, are a group selected from: C₁-C₆ alkyls, phenyl, benzyl, the groups -COR⁴ and R⁴ is a group selected from: C₁-C₆ alkyls, phenyl, benzyl,
R³ is a group selected from: H, C₁-C₆ alkyls, phenyl, benzyl, a peptide chain of amino acids comprising from 1 to 30 amino acids,
* is the L optical configuration,
R⁵ is a group selected from: H, C₁-C₆ alkyls, phenyl, benzyl, the groups -CO-R⁶ and R⁶ is a group selected from: C₁-C₆ alkyls, phenyl, benzyl,
not including the following compounds in which:
A is -CH=CH-, x₁ = 1, x₂ = 1, and
- R¹ = R² = CH₃CO-, R³ = CH₃, R⁵ = CH₃CO-, or
- R¹ = R² = CH₃CO-, R³ = H, R⁵ = H, or
- R¹ = R² = CH₃CO-, R³ = tBu, R⁵ = tBu.

12. Method according to any one of claims 1 to 9 for preparing a compound having the formula (Ic) given below: 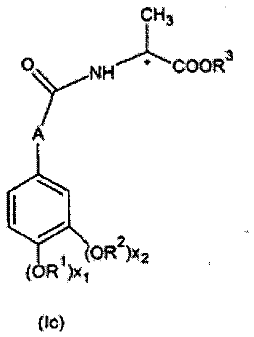 in which
A is a group selected from -CH=CH- and -CH₂-CH₂-,
x₁ is an integer selected from 0 and 1,
x₂ is an integer selected from 0 and 1,
R¹, R², independently of one another, are a group selected from: C₁-C₆ alkyls, phenyl, benzyl, the groups -CO-R⁴ and R⁴ is a group selected from: C₁-C₆ alkyls, phenyl, benzyl,
R³ is a group selected from: H, C₁-C₆ alkyls, phenyl, benzyl, a peptide chain of amino acids comprising from 1 to 30 amino acids,
* is the L or D optical configuration,
not including the cases in which:
* is the L optical configuration and
A is -CH=CH-, and
- x₁ = x₂ = 0, R³ = tBu.

13. Method according to any one of claims 1 to 9 for preparing a compound having the general formula (Id) given below: 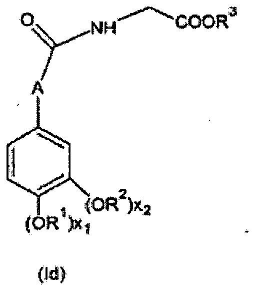 in which
A is a group selected from -CH=CH- and -CH₂-CH₂-,
x₁ is an integer selected from 0 and 1,
x₂ is an integer selected from 0 and 1,
R¹, R², independently of one another, are a group selected from: C₁-C₆ alkyls, phenyl, benzyl, the groups -CO-R⁴ and R⁴ is a group selected from: C₁-C₆ alkyls, phenyl, benzyl,
R³ is a group selected from: H, C₁-C₆ alkyls, phenyl, benzyl, a peptide chain of amino acids comprising from 1 to 30 amino acids,
not including the cases in which:
A is -CH=CH- and
- x₁ = 1, x₂ = 0, R¹ = CH₃, R³ = H
- x₁ = 1, x₂ = 0, R¹ = CH₃, R³ = C₂H₅
- x₁ = x₂ = 0, R³ = H.
